# EUROPÄISCHE PATENTANMELDUNG

(11) **EP 2 710 985 A2**
(43) Veröffentlichungstag der Anmeldung: **26.03.2014**
(21) Anmeldenummer: 13180502.0
(22) Anmeldetag: 15.08.2013
(51) Int. Cl.: A61F 2/95, A61F 2/966, A61F 2/24

(54) **Implantat, System aus einem Implantat und einem Katheter sowie Verfahren zur Herstellung eines solchen Systems**

(30) Priorität: 20.09.2012 US 201261703278 P
(71) Anmelder: Biotronik AG, 8180 Bülach (CH)
(72) Erfinder: Bardill, Anita, 8006 Zürich (CH); Blaser, Adrian, 8032 Zürich (CH); Jakob, Aldo, 8193 Eglisau (CH)
(74) Vertreter: Galander, Marcus

(57) **Zusammenfassung**

Die vorliegende Erfindung betrifft ein Implantat (10, 10', 10", 10'"), insbesondere eine intraluminale Endoprothese, mit einer durchbrochenen, hohlzylinder- und/oder hohlkegelförmigen Grundstruktur (11), wobei die Grundstruktur (11) einen komprimierten Zustand oder einen expandierten Zustand einnehmen kann. Zur Vereinfachung der Endmontage durch den Arzt ist zusätzlich ein erster rohrförmiger Schaft (16, 16') vorgesehen, auf dessen Außenseite die Grundstruktur (11) zumindest abschnittsweise im komprimierten Zustand angeordnet ist, wobei der erste Schaft (16, 16') einen ersten Verbindungsabschnitt (20, 23) aufweist, mit welchem der erste Schaft (16, 16') mit dem Innenschaft (32, 32', 32a, 32b) eines Katheters (30, 30', 30") verbindbar ist. Die Erfindung betrifft ferner ein entsprechendes System aus einem derartigen Implantat und einem Katheter (30, 30', 30") sowie ein Verfahren zur Herstellung eines solchen Systems.

## Beschreibung

Die vorliegende Erfindung betrifft ein Implantat, insbesondere eine intraluminale Endoprothese, mit einer durchbrochenen, hohlzylinderförmigen und/oder hohlkegelförmigen Grundstruktur, wobei die Grundstruktur einen komprimierten Zustand und einen expandierten Zustand einnehmen kann. Die Erfindung betrifft ferner ein System zum Einbringen eines Implantats, vorzugsweise einer intraluminalen Endoprothese, in einen Körperhohlraum bestehend aus dem vorstehend beschriebenen Implantat und einem Katheter. Außerdem betrifft die Erfindung ein Verfahren zur Herstellung eines derartigen Systems.

Medizinische Implantate, insbesondere intraluminale Endoprothesen, für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stents (vaskuläre Stents, einschließlich Stents für die Anwendung im Bereich des Herzens und Herzklappenstents, z.B. Mitralklappen-Stent, Pulmonalklappenstent) und Gallengang-Stents), Endoprothesen zum Verschließen von persistierenden Foramen ovale (PFO), Stentgrafts zur Behandlung von Aneurysmen, Endoprothesen zum Verschließen eines ASD (Vorhofscheidewanddefekt, atrial septal defect) sowie Prothesen im Bereich des Hart- und Weichgewebes zu verstehen. Ein derartiges Implantat wird häufig mittels eines Katheters in das zu behandelnde Organ oder Gefäß eingesetzt.

Stents und andere endovaskuläre Endoprothesen weisen eine durchbrochene hohlzylinderförmige (rohrförmige) und/oder hohlkegelförmige Grundstruktur auf, die an beiden Längsenden offen ist, wobei die Grundstruktur häufig aus einer Vielzahl von Stegen zusammengesetzt ist. In einer derartige Grundstruktur können z.B. bei einem Herzklappenstent auf der Innenseite Klappensegel, beispielsweise drei Klappensegel, angeordnet sein, welche die Herzklappe ausbilden und aus einem Kunststoff oder einem biologischen Material, z.B. Schweinepericard, bestehen können. Der Stent trägt die Herzklappe und verankert diese im Herzen.

Katheter sind Röhrchen oder Schläuche verschiedenen Durchmessers, die in den jeweiligen zu behandelnden Körperhohlraum eingeführt werden können. Bei der Behandlung mit einem Katheter wird zuerst ein Führungsdraht und ggf. ein Führungskatheter in das zu behandelnde Organ oder Gefäß eingeführt. Anschließend wird der Katheter entlang des Führungsdrahts bis zu der zu behandelnden Stelle des Organs oder Gefäßes vorgeschoben, so dass das auf dem Katheter angeordnete Implantat im Bereich der zu behandelnden Stelle des Organs oder Gefäßes platziert ist. Danach wird das Implantat von dem Katheter abgekoppelt und ggf. dilatiert. Der Katheter wird dann entfernt. Gleichzeitig oder anschließend wird auch der Führungsdraht aus dem Organ oder Gefäß zurückgezogen. Für das Einbringen eines Stents wird häufig ein Katheter verwendet, welcher einen Innenschaft und einen Außenschaft besitzt.

Stents und andere Implantate nehmen üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels eines Katheters in das zu behandelnde Gefäß oder Organ durch enge Gefäße hindurch eingeführt und an der zu behandelnden Stelle positioniert werden. Hierfür wird das Implantat beispielsweise auf den Ballon eines Katheters aufgecrimpt und so in den komprimierten Zustand überführt. Am Ort der Behandlung wird das Implantat dann beispielsweise mittels des Ballons des Katheters dilatiert und nimmt dann den expandierten Zustand ein, in dem das Implantat in dem Gefäß oder Organ verbleibt und dort festgelegt ist, nachdem der Katheter wieder aus dem Körper des Behandelten herausgezogen wurde. Alternativ nimmt ein Implantat in dem Fall, in dem seine Grundstruktur aus einem selbstexpandierenden Material (z.B. Nitinol) besteht, den komprimierten Zustand durch Komprimierung unterhalb der Übergangstemperatur und den expandierten Zustand oberhalb der Übergangstemperatur ein.

Aus der Druckschrift US 8,029,564 B2 ist eine Herzklappenprothese und eine Umlenkeinrichtung bekannt. Das System beinhaltet ferner ein Band, das durch die freien Enden der Stentstangen, welche die Herzklappe tragen, hindurch geführt wird. Mittels dieses Bandes können die Stentstangen nach innen umgebogen werden, um einen geschlossenen Zustand zu erreichen. Dieses System eignet sich jedoch nicht dafür, nichtinvasiv mittels eines Katheters zu der behandelnden Stelle transferiert zu werden, da es im Fußbereich zu voluminös ist.

Bei Herzklappenstents, die mittels eines Katheters zu der zu behandelnden Stelle transportiert werden können, z.B. die CoreValve® Transkatheter Herzklappe der Fa. Medtronic GmbH, ist vor dem nicht-invasiven Eingriff zunächst eine komplizierte Montage des Stents notwendig, um diesen auf den Katheter zu montieren. Hierfür werden derzeit fünf verschiedene Hilfstools eingesetzt. Die Prozedur, die in der Regel durch den Arzt durchgeführt wird, ist sehr aufwändig und besitzt darum eine hohe Fehleranfälligkeit.

Ferner besteht das Problem, dass bei den bekannten Systemen die Stenthaltekraft ungenügend sein kann. Dies bedeutet, dass die Enden eines auf dem Katheter angeordneten Stents in einer Kurve eines Gefäßes, durch welches das System auf dem Weg zu dem zu behandelnden Körperhohlraum hindurch geführt wird, aus der Fixierung herausspringen kann. Wenn dies eintritt, können sich Ungenauigkeiten bei der Platzierung des Stents an dem zu behandelnden Ort ergeben bzw. es kann ein erneutes Crimpen oder Einfangen des Stents durch den Katheter notwendig werden, was äußerst schwierig zu bewerkstelligen ist.

Die Aufgabe besteht somit darin, ein Implantat zu schaffen, welches eine Vereinfachung der Stentmontage ermöglicht. Ferner soll der Stent besser auf dem Katheter fixiert werden, um ein Herausspringen aus der Verankerung zu vermeiden. Die Aufgabe besteht außerdem darin, ein entsprechendes System aus Katheter und Implantat zu schaffen. Ferner besteht die Aufgabe darin, ein Verfahren zur Herstellung eines Systems aus Katheter und intraluminaler Endoprothese anzugeben, welches einfach durchführbar ist und ein geringes Fehlerrisiko beinhaltet.

Die obige Aufgabe wird durch ein Implantat mit den Merkmalen des Anspruchs 1 gelöst. Insbesondere ist bei dem erfindungsgemäßen Implantat zusätzlich ein erster rohrförmiger Schaft vorgesehen, auf dessen Außenseite die Grundstruktur des Implantats zumindest abschnittsweise im komprimierten Zustand angeordnet ist, wobei der erste Schaft einen ersten Verbindungsabschnitt aufweist, mit welchem der erste Schaft mit dem Innenschaft eines Katheters verbindbar ist.

Das erfindungsgemäße Implantat enthält in Form des ersten Schafts gewissermaßen bereits einen Teil des Innenschafts des Katheters, mit dem das Implantat dann zu der zu behandelnden Stelle transportiert wird. Diese Funktion füllt der erste Schaft jedoch erst dann aus, wenn der Innenschaft des Katheters mit dem ersten Schaft verbunden ist. Die Erfinder haben insbesondere erkannt, dass bei der hierdurch erfindungsgemäß realisierten Vormontage des Implantats auf einem entkoppelten distalen Innenschaft-Abschnitt (entspricht dem ersten Schaft) ein Teil der komplexen und schwierigen Arbeitsschritte bei der Initialfixierung des Implantats nicht mehr durch den Arzt durchgeführt werden muss, sondern stattdessen unter standardisierten Bedingungen bereits bei dem Hersteller erledigt werden kann. Hierdurch wird das Risiko, dass sich ein vom Arzt nicht korrekt auf dem Katheter fixiertes Implantat schwer entfaltet, minimiert. Somit werden bei der Montage beim Arzt die Anzahl der Arbeitsschritte verringert und die Arbeitsschritte vereinfacht sowie anwenderfreundlicher gestaltet. Das vormontierte Implantat kann ggf. in vorteilhafter Weise bis zu seinem Einsatz problemlos in der notwendigen Flüssigkeit gelagert werden. Vorzugsweise wird bei einem Herzklappenstent der Abschnitt, der in vormontiertem Zustand bei der Anordnung auf dem ersten Schaft komprimiert wird, am proximalen Ende des Stents ohne Klappensegel angeordnet, so dass gewährleistet ist, dass das Herklappenmaterial (Pericard oder Kunststoff) im entfalteten Zustand gelagert werden kann.

Die Verbindung mit dem Innenschaft des Katheters durch den ersten Verbindungsabschnitt des ersten Schafts kann mittels einer beliebigen kraft-, form- und/oder stoffschlüssigen Verbindung erfolgen, bspw. mit einem Bajonett-Verschluss, einem Gewinde oder einem Klebstoff. Die Verbindung kann lösbar oder nicht lösbar ausgeführt werden.

In einem Ausführungsbeispiel ist zu dem Implantat ein zweiter, zumindest abschnittsweise rohrförmigen Schaft vorgesehen, der die Grundstruktur umgebend angeordnet ist, vorzugsweise an ihrem proximalen Ende. Vorzugsweise umgibt der zweite Schaft die Grundstruktur in dem Abschnitt, in dem das Implantat im komprimierten Zustand auf dem ersten Schaft angeordnet ist. Mittels des zweiten Schafts erfolgt die Fixierung des Implantats auf dem ersten Schaft durch den Hersteller bzw., nach der Verbindung des ersten Schafts mit dem Innenschaft des Katheters, auf dem Katheter. Hierdurch wird eine erhöhte Implantat-Haltekraft erreicht, so dass das Implantat in einer Gefäßkurve besser auf dem Katheter fixiert ist und nicht herausspringt. Dadurch ist das Implantat präziser am Ort der Behandlung platzierbar und ein erneutes Crimpen oder Einfangen des Implantats ist nicht notwendig. Der zweite Schaft kann alternativ auch zum Vorcrimpen der Grundstruktur des Implantats vor der Fixierung mit einem Außenschaft verwendet werden.

In einer Weiterbildung der Erfindung ist vorgesehen, dass zusätzlich Mittel zur Fixierung des zweiten Schafts in proximaler Richtung vorgesehen sind, beispielsweise ein proximal angeordneter Deckel oder ein am proximalen Ende des zweiten Schafts angeordneter, in radialer Richtung vorstehender Pin. Durch das Grundgitter des Implantats wird nämlich auf den zweiten Schaft eine Kraft ausgeübt, welche zu einer Verschiebung des Schafts in proximaler Richtung führt. Der zweite Schaft kann hierdurch von dem Implantat rutschen. Die Mittel zur Fixierung, z.B. ein Deckel oder ein in radialer Richtung vorstehender Pin, der am proximalen Ende des zweiten Schafts angeordnet ist, wirken der von dem Grundgitter auf den zweiten Schaft ausgeübten Kraft entgegen. Weitere reversible form- oder kraftschlüssige Techniken kommen für diese Fixierung ebenfalls in Frage.

Es ist in einem weiteren Ausführungsbeispiel vorgesehen, dass der zweite Schaft einen konischen Abschnitt aufweist, der vorzugsweise am distalen Ende des zweiten Schafts angeordnet ist. Durch diesen Abschnitt kann bei der Montage des Implantats das auf der distalen Seite des zweiten Schafts angeordnete Teil des Grundgitters besser gecrimpt werden.

Es ist weiter von Vorteil, wenn der zweite Schaft einen Verbindungsabschnitt aufweist, mit dem der zweite Schaft mit einem rohrförmigen Außenschaft oder einem rohrförmigen Fixierschaft (Hilfsschaft) des Katheters verbindbar ist. Die Verbindung mit dem Außenschaft oder dem Fixierschaft des Katheters kann prinzipiell durch jede beliebige kraft, form- oder stoffschlüssige Verbindung gebildet werden und sie kann entweder lösbar oder nicht lösbar ausgestaltet sein. Bei diesem Ausführungsbeispiel erfolgt das Crimpen während der Montage des Implantats durch Verschieben des Außenschafts in distaler Richtung. Der ggf. zusätzlich vorgesehene, zwischen Innenschaft und Außenschaft angeordnete Fixierschaft dient dazu, das Implantat an der gewünschten Stelle auf dem Innenschaft des Katheters bzw. dem ersten Schaft losgelöst vom Außenschaft, d.h. eigenständig zu halten, auch wenn bei dem Transportieren zu der zu behandelnden Stelle im Körper Kurven in den Gefäßen zu überwinden sind. Die Fixierung der Grundstruktur durch den Fixierschaft kann nach erfolgreicher Platzierung des Implantats an der gewünschten Stelle z.B. durch den behandelnden Arzt in einem separaten Schritt, d.h. losgelöst vom Außenschaft, gelöst werden.

Bei der Montage des Implantats auf dem Katheter besteht ferner das Problem, dass die Spitze des Katheters einen so großen Außendurchmesser aufweisen kann, dass ein Hilfsrohr z.B. in Form eines zweiten Schafts zum Crimpen nicht über die Spitze geschoben werden kann. In diesem Fall ist es von Vorteil, wenn der Innenschaft des Katheters zweiteilig gestaltet ist und entsprechend der erste Schaft, vorzugsweise an seinem distalen Ende, einen zweiten Verbindungsabschnitt aufweist, mit welchem der erste Schaft mit der distalen Spitze des Innenschafts eines Katheters verbindbar ist. Dann erfolgt die Verbindung des ersten Schafts mit der Katheterspitze bzw. dem zweiten Teil des Innenschafts erst nach dem Crimpen. Auch die Verbindung zwischen dem zweiten Verbindungsabschnitt des ersten Schafts und dem zweiten Teil des Innenschafts des Katheters kann wieder als jede beliebige kraft-, form- und/oder stoffschlüssige Verbindung ausgebildet sein, welche lösbar oder nicht lösbar ausgeführt werden kann.

Die obige Aufgabe wird mit den oben angegebenen Vorteilen außerdem durch ein System zum Einbringen eines Implantats gelöst, bei dem der Innenschaft des Katheters einen, vorzugsweise an seinem distalen Ende angeordneten Verbindungsabschnitt aufweist, mit dem der Innenschaft mit dem ersten Schaft des Implantats verbindbar ist. Hierbei entspricht das Implantat einer der oben angegebenen Ausführungsformen.

Die Vorteile des erfindungsgemäßen Systems sind oben bereits im Zusammenhang mit dem erfindungsgemäßen Implantat erläutert worden.

In einem bevorzugten Ausführungsbeispiel weist der Außenschaft des Katheters oder der Fixierschaft des Katheters jeweils einen, vorzugsweise an dem jeweiligen distalen Ende angeordneten, Verbindungsabschnitt auf, mit dem der Außenschaft bzw. der Fixierschaft mit dem zweiten Schaft des Implantats verbindbar ist. Wie oben bereits erläutert wurde, kann der Fixierschaft des Katheters zur eigenständigen Fixierung des Implantats verwendet werden.

Weiter bevorzugt ist der Innenschaft des Katheters zweiteilig ausgebildet, wobei der erste Teil durch einen proximalen Abschnitt und der zweite Teil durch einen distalen Abschnitt des Innenschafts gebildet ist. Der distale Abschnitt enthält insbesondere die Katheterspitze, welche hinsichtlich des Einführens des erfindungsgemäßen Systems in den Körper von besonderer Bedeutung ist, da sie nach dem Führungsdraht das erste Element des Katheters darstellt, welches in den Körper eindringt und sich in führender Position während der Bewegung des Katheters durch den Körper befindet.

Die obige Aufgabe wird außerdem gelöst durch ein Verfahren, bei dem vor dem Einbringen des Systems in einen Körperhohlraum eines Menschen oder Tiers der erste Verbindungsabschnitt des ersten Schafts mit dem gegenüber liegenden Verbindungsabschnitt des Innenschafts des Katheters verbunden wird. Dieses Verfahren stellt eine einfache Möglichkeit zur Herstellung eines Systems bestehend aus einem Katheter und einem Implantat dar. Das erfindungsgemäße Verfahren beinhaltet auf Seiten des Arztes weniger Arbeitsschritte als das herkömmliche Verfahren und erleichtert damit insbesondere dessen Tätigkeit vor einer Implantation. Nach der Verbindung des ersten Schafts mit dem Innenschaft des Katheters kann danach der Grundkörper beispielsweise mittels eines zweiten Schafts, der nach distal vorgeschoben wird, vorgecrimpt werden. Der Außenschaft, welcher ggf. unmittelbar proximal hinter dem zweiten Schaft folgt, wird anschließend über das Implantat geführt. Danach wird der zweite Schaft distal über die Katheterspitze geschoben und vom Instrument entfernt. Weitere Ausführungsformen werden unten in den Ausführungsbeispielen erläutert.

Bei einer zweiteiligen Ausbildung des Innenschafts wird vor dem Einbringen des Systems in einen Körperhohlraum eines Menschen oder Tiers der erste Verbindungsabschnitt des ersten Schafts mit dem gegenüber liegenden Verbindungsabschnitts des ersten Teils des Innenschafts des Katheters und der zweite Verbindungsschaft des ersten Schafts mit dem gegenüberliegenden Verbindungsabschnitt des zweiten Teils des Innenschafts des Katheters verbunden. Wie bereits oben erläutert wurde, ist diese Ausführungsform insbesondere dann von Bedeutung, wenn ein zweiter Schaft zum Vorcrimpen des Implantats derart dimensioniert ist, dass der jeweilige Schaft nicht über die Katheterspitze geschoben werden kann. In diesem Fall wird nach der Verbindung des ersten Teils des Innenschafts mit dem ersten Schaft zuerst das Grundgitter mittels zweiten Schafts vorgecrimpt und anschließend vom Implantat entfernt. Danach wird der zweite Teil des Innenschafts mit dem zweiten Verbindungsabschnitt des ersten Schafts verbunden.

Es ist ferner von Vorteil, wenn der Verbindungsabschnitt des zweiten Schafts mit dem gegenüber liegenden Verbindungsabschnitt des Außenschafts des Katheters oder mit dem gegenüber liegenden Verbindungsabschnitt des Fixierschafts des Katheters verbunden wird. Im ersten Fall wird der Außenschaft des Katheters sowie der mit diesem vorab verbundene zweite Schaft zum Crimpen des Grundgitters verwendet, während im zweiten Fall eine Fixierung des Grundgitters durch den zweiten Schaft erfolgt.

Weitere Ziele, Merkmale, Vorteile und Anwendungsmöglichkeiten der Erfindung ergeben sich aus der nachfolgenden Beschreibung von Ausführungsbeispielen zu der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: ein erstes Ausführungsbeispiel eines erfindungsgemäßen Systems mit einem ersten Ausführungsbeispiel eines erfindungsgemäßen Implantats im Querschnitt,
- Fig. 2: ein zweites Ausführungsbeispiel eines erfindungsgemäßen Implantats im Querschnitt,
- Fig. 3: ein drittes Ausführungsbeispiel eines erfindungsgemäßen Implantats im Querschnitt,
- Fig. 4: ein zweites Ausführungsbeispiels eines erfindungsgemäßen Systems mit einem vierten Ausführungsbeispiel eines erfindungsgemäßen Implantats im Querschnitt,
- Fig. 5: ein drittes Ausführungsbeispiels eines erfindungsgemäßen Systems mit einem fünften Ausführungsbespiel eines erfindungsgemäßen Implantats im Querschnitt und
- Fig. 6: ein viertes Ausführungsbeispiel eines erfindungsgemäßen Systems mit einem sechsten Ausführungsbeispiel eines erfindungsgemäßen Implantats, ebenfalls jeweils im Querschnitt.

Die Figuren zeigen die Ausführungsbeispiele schematisch und vereinfacht und stellen insbesondere die Details dar, die wichtig sind, um die Erfindung zu verstehen. Für die Erfindung unbedeutende Details wurden teilweise weggelassen. Weiterhin bedeutet die Bezeichnung "distales Ende" im Zusammenhang mit der vorliegenden Erfindung das Ende des Implantats oder des Katheters, das während dem Einbringen des Implantats in den Körper von dem behandelnden Arzt weg zeigt, während das "proximale Ende" zu der den Katheter bedienenden Person hin zeigt.

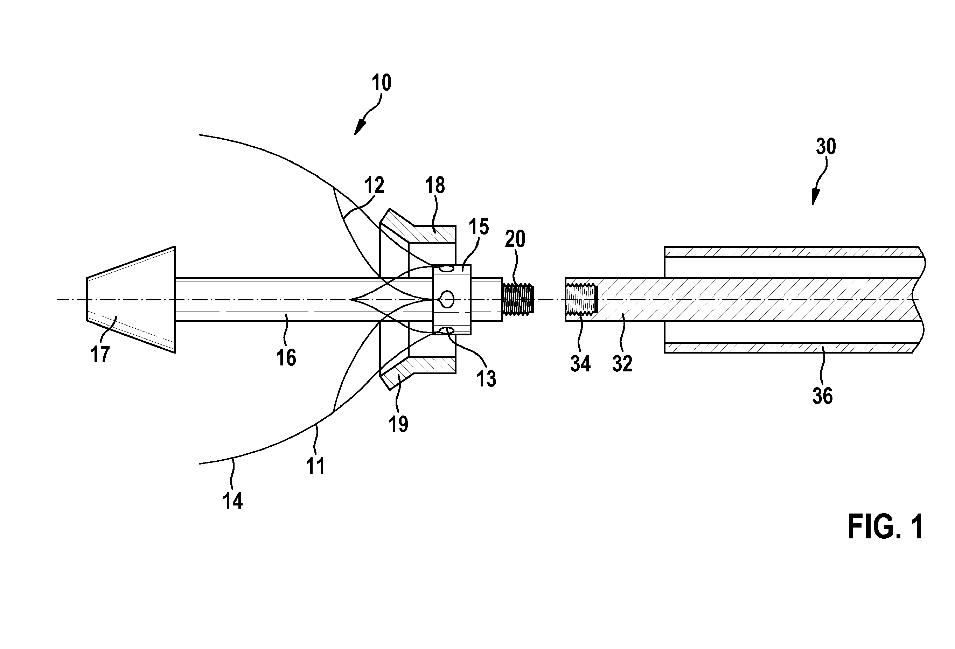

In Fig. 1 wird im noch nicht montierten Zustand das erste Ausführungsbeispiel eines erfindungsgemäßen Systems mit einem erfindungsgemäßen Implantat, welches als Herzklappenstent 10 ausgebildet ist, und einem Katheter 30 dargestellt. Das erfindungsgemäße Implantat 10 weist eine Grundstruktur 11 auf, welche durchbrochen ausgebildet ist und vorzugsweise aus einer Vielzahl von Stegen besteht, die eine Maschenstruktur ergeben. Die an beiden Enden offene Grundstruktur 11 besitzt an ihrem proximalen Ende 13 einen hohlzylinderförmigen Abschnitt und an ihrem distalen Ende 14 einen hohlkegelförmigen Abschnitt. In dem durch die Grundstruktur 11 eingeschlossenen Innenbereich weist der Herzklappenstent 30 vorzugsweise drei Klappensegel 12 auf, welche die Herzklappe ausbilden und jeweils beispielsweise aus einem biologischen Material, z.B. Schweinepericard, bestehen. Die Klappensegel können alternativ aus einem Kunststoff ausgebildet sein. An dem proximalen Ende 13 des Herzklappenstents ist die Grundstruktur 11 komprimiert, während die Grundstruktur 11 an ihrem distalen Ende 14 im expandierten Zustand vorliegt. Hierbei ist die Grundstruktur 11 an ihrem äußersten proximalen Ende 13 in einem Ring 15 befestigt. Hierfür sind am äußersten proximalen Ende 13 an der Grundstruktur 11 ösenförmige Ringe oder T-förmige Elemente ausgebildet, welche in entsprechende Aussparungen des Rings 15 passen.

Die Grundstruktur 11 des Herzklappenstents 10, welche beispielsweise aus einem selbstexpandierenden Material (z.B. Nitinol) besteht, ist auf einem ersten Schaft 16 angeordnet, welcher durch einen hohlzylinderförmigen Schlauch gebildet wird. Im proximalen Abschnitt des ersten Schafts 16 ist ein Ring 15 mit Aussparungen fixiert. Die Anbindung der Grundstruktur 11 an den ersten Schaft 16 erfolgt dadurch, dass die ösenförmigen Ringe oder T-förmigen Elemente am äußersten proximalen Ende 13 der Grundstruktur 11 in die entsprechenden Aussparungen des Rings 15 gekoppelt werden. Am distalen Ende des ersten Schafts 16 ist ein konisch verlaufender Abschnitt 17 vorgesehen, welcher nach Verbindung des ersten Schafts 16 mit dem Innenschaft 22 des Katheters 20 die Spitze des Katheters 30 ausbildet. Hierfür ist der Konus so angeordnet, dass das Ende mit dem geringeren Durchmesser an dem distalen Ende des ersten Schafts 16 liegt.

An dem proximalen Ende 13 der Grundstruktur 11 ist ferner ein auf der Grundstruktur 11 angeordneter zweiter Schaft 18 vorgesehen, welcher an seinem distalen Ende einen konischen Abschnitt 19 aufweist. Der zweite Schaft 18 hindert beispielsweise die aus einem selbstexpandieren Material bestehende Grundstruktur 11 eines Herzklappenstents 10 daran, sich zu öffnen, wodurch diese sich vom ersten Schaft 16 ablösen würde. An seinem proximalen Ende besitzt der erste Schaft 16 einen ersten Verbindungsabschnitt 20, an welchem beispielsweise ein Außengewinde vorgesehen ist.

Bei der Montage des Systems wird der Katheter 30, d.h. insbesondere ein Verbindungsabschnitt 34 des Innenschafts 32, der z.B. ein Innengewinde aufweist, mit dem Außengewinde des ersten Verbindungsabschnitts 20 des ersten Schafts derart in Eingriff gebracht, dass der Innenschaft 32 des Katheters 30 mit dem ersten Schaft 16 des Implantats verbunden ist. Anschließend wird der zweite Schaft 18 in distaler Richtung verschoben, um die Grundstruktur 11 mit den Klappensegeln 12 zu crimpen. Danach wird der Außenschaft 36 über den gecrimpten Herzklappenstent 10 ebenfalls in distaler Richtung bewegt und der zweite Schaft 18 von dem Instrument entfernt, wobei hierzu der zweite Schaft 18 über den konischen Abschnitt 17 des ersten Schafts 16 geschoben wird.

Als alternative Möglichkeiten, den ersten Schaft 16 und den Innenschaft 32 des Katheters 30 in den jeweiligen Verbindungsabschnitten 20, 43 miteinander zu verbinden, kommen z.B. auch die Verwendung eines Bajonettverschlusses oder von Klebstoff (Sekundenkleber) in Frage.

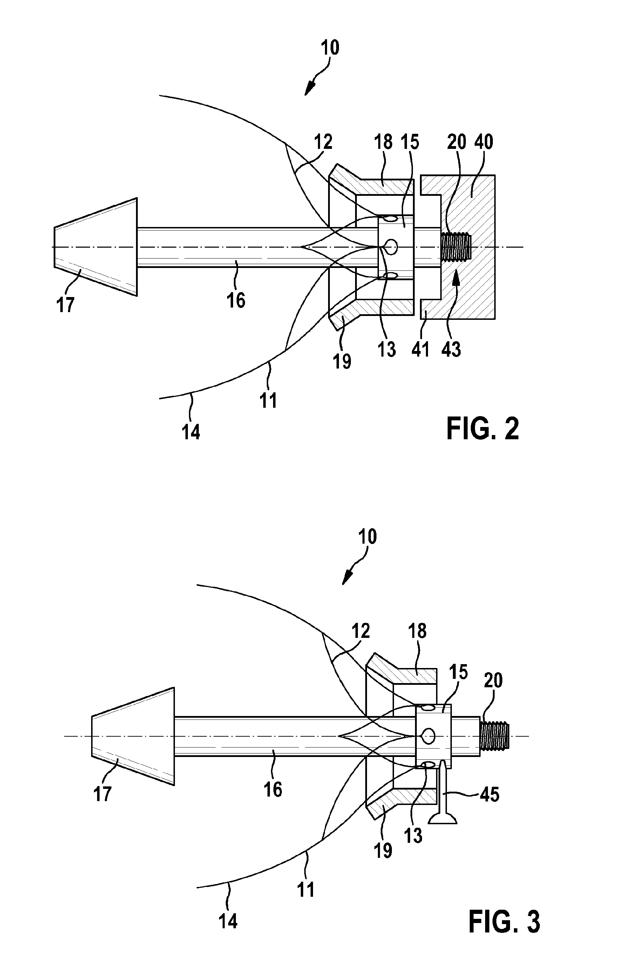

Um zu verhindern, dass der zweite Schaft 18 in proximaler Richtung von der Grundstruktur 11 abfällt, ist bei der in Fig. 2 gezeigten zweiten Ausführungsform eines erfindungsgemäßen Implantats zusätzlich ein Deckel 40 vorgesehen, welcher vorzugsweise mittels eines in distaler Richtung vorstehenden ringförmigen Abschnitts 41, der dem zweiten Schaft 18 gegenüber liegt, die Verschiebung des zweiten Schafts 18 in proximaler Richtung verhindert. Ferner weist der Deckel 40 einen Gewindeabschnitt 43 auf, welcher ein Innengewinde vorsieht. Das Innengewinde des Gewindeabschnitts 43 kann mit dem Außengewinde des ersten Verbindungsabschnitts 20 verbunden werden.

Alternativ kann, wie in Fig. 3 gezeigt, am proximalen Ende 13 der Grundstruktur 11 ein in radialer Richtung abstehender Pin 45 vorgesehen sein, welcher beispielsweise in einer entsprechenden Öffnung des Grundgitters 11 oder des Rings 15 befestigt ist. Dieser Pin 45 verhindert aufgrund seiner proximalen Anordnung zu dem zweiten Schaft 18 die Verschiebung des zweiten Schafts 18 in proximaler Richtung.

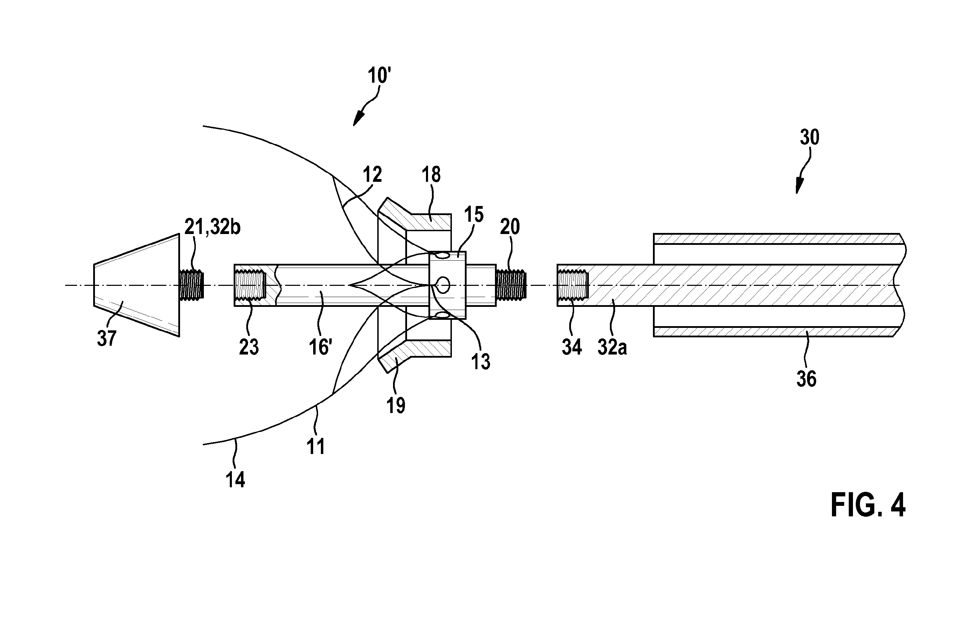

Fig. 4 zeigt das zweite Ausführungsbeispiel eines erfindungsgemäßen Systems, das in seinem Aufbau im Wesentlichen dem in Fig. 1 dargestellten ersten Ausführungsbeispiel entspricht. Allerdings ist, wie Fig. 4 zeigt, der Innenschaft zweiteilig ausgebildet, mit einem proximalen ersten Teil 32a und einem distalen zweiten Teil 32b. Der zweite Teil 32b des Innenschafts enthält auch einen konischen Abschnitt 37, der nach der Montage des Systems die Katheterspitze bildet. Entsprechend weist der erste Schaft 16 des erfindungsgemäßen Implantats einen zweiten Verbindungsabschnitt 23 auf, der beispielsweise ein Innengewinde enthält. Der zweite Teil 32b des Innenschafts sieht entsprechend an seinem proximalen Ende einen Verbindungsabschnitt 21 mit einem Außengewinde vor. Alternativ kann der zweite Teil 32b des Innenschafts des Katheters 30 mit dem ersten Schaft 16' z.B. auch mittels eines Bajonettverschlusses oder mittels Klebstoff verbunden werden.

Dieses Ausführungsbeispiel ist insbesondere für den Fall vorteilhaft, wenn der zweite Schaft 18 so dimensioniert ist, dass er nicht über die Katheterspitze (konischer Abschnitt 37) geschoben werden kann. Entsprechend wird bei dem in Fig. 4 dargestellten Ausführungsbeispiel zunächst die Grundstruktur 11 mit dem Klappensegel 12 mittels des zweiten Schafts 18 vorgecrimpt und der Außenschaft 36 danach über das Grundgitter 11 geschoben. Dann wird der zweite Teil 32b des Innenschafts mit dem ersten Schaft 16` des Herzklappenstents 10 wie oben beschrieben verbunden.

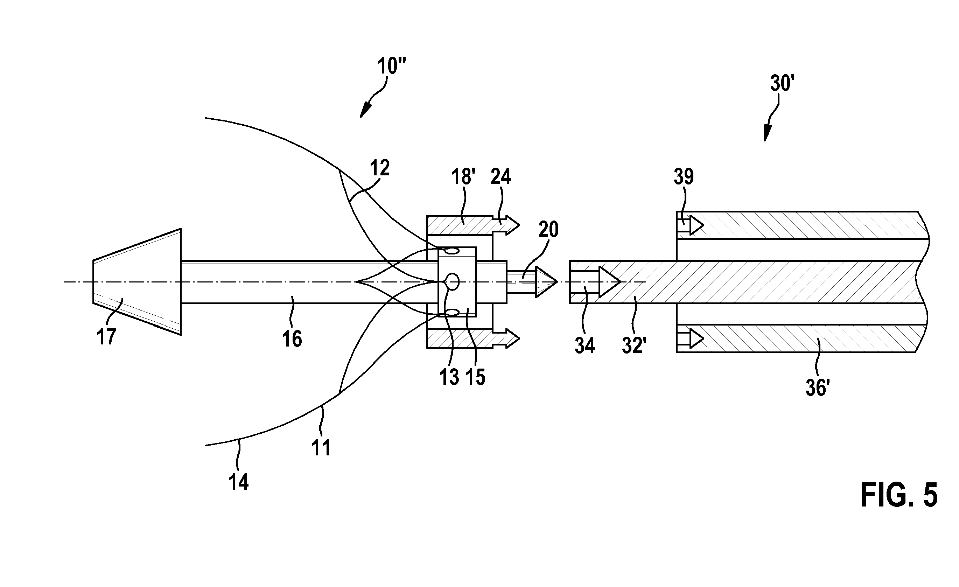

Das in Fig. 5 dargestellte dritte Ausführungsbeispiel eines erfindungsgemäßen Systems zeigt den Fall, bei dem der zweite Schaft 18' mit dem Außenschaft 36' und der erste Schaft 16 mit dem Innenschaft 32' gekoppelt werden. Der erste Schaft 16 und der zweite Schaft 18' sind zunächst von der Freisetzeinheit (Katheter) entkoppelt und bilden nach der Kopplung durch den Arzt gewissermaßen distale Teile des Innenschafts 32' bzw. des Außenschafts 36' und somit distale Teile der Freisetzeinheit (Katheter). In diesem Fall braucht der Herzklappenstent 10" nicht durch einen weiteren Schaft fixiert zu werden, sondern kann durch den proximalen Außenschaft 36' festgelegt werden. Der zweite Schaft 18' weist an seinem proximalen Ende einen Verbindungsabschnitt 24 auf, der mit dem distalen Verbindungsabschnitt 39 des Außenschafts 36' verbunden werden kann. Zudem kann der Verbindungsabschnitt 20 des ersten Schafts 16 mit dem distalen Verbindungsabschnitt 34 des Innenschafts 32' verbunden werden.

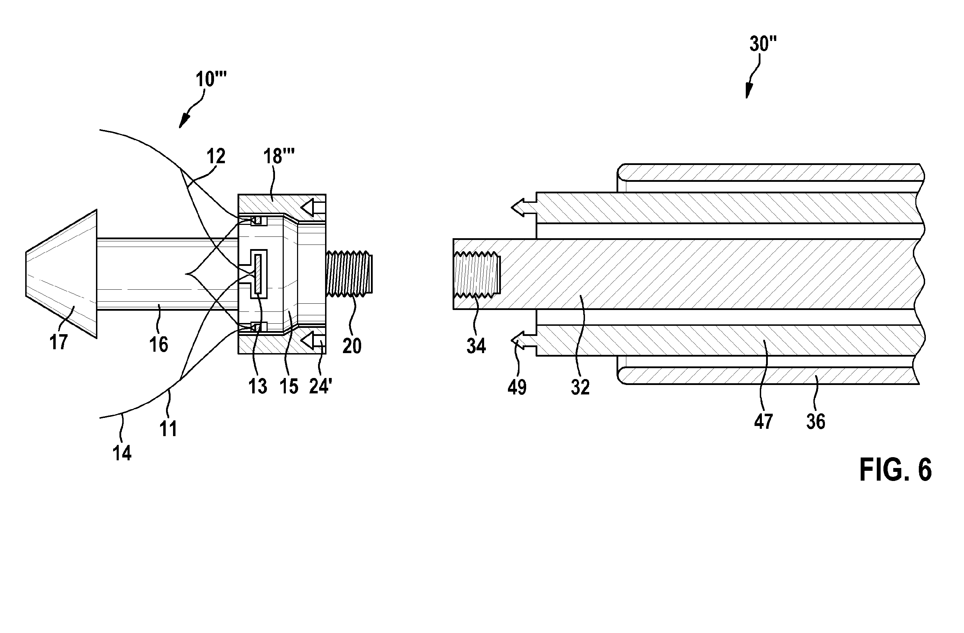

Das in Fig. 6 dargestellte Ausführungsbeispiel eines erfindungsgemäßen Systems beschreibt eine konstruktive Lösung, bei dem der Katheter 30" drei Schäfte aufweist, nämlich einen Innenschaft 32, einen Außenschaft 36 und einen dazwischen liegenden Fixierschaft (Hilfsschaft) 47. Dieses Ausführungsbeispiel beinhaltet die Möglichkeit, dass das proximale Ende des Herzklappenstents 10'" durch den von der Freisetzeinheit entkoppelten Fixierschaft 47 über einen kraft- oder formschlüssigen Mechanismus auf dem ersten Schaft 16 fixiert wird.

Bei der Kopplung des ersten Schafts 16 des Implantats 10"' an den Katheter 30" über einen im Zusammenhang mit Fig. 1 bereits beschriebenen Mechanismus wird in diesem Fall gleichzeitig der Fixierschaft 47 an den zweiten Schaft 18" gekoppelt und mit diesem verbunden. Sind Innenschaft 32 und Hilfsschaft 47 gekoppelt, wird das Grundgitter 11 durch Vorschieben des Außenschafts 36 in distale Richtung gecrimpt. Der Herzklappenstent 10"' wird an seinem proximalen Ende durch den zweiten Schaft 18" und nach der Montage durch den Fixierschaft 47 am Instrument so lange fixiert, bis der Arzt, beispielsweise nach der Platzierung des Stents 10"' in dem gewünschten Bereich des Körpers des Behandelten, die Fixierung löst.

Dieses Ausführungsbeispiel ist besonders vorteilhaft hinsichtlich der Fixierung des Stents, da das Stentende in der Kurve nicht mehr aus der Fixierung springen kann. Hierdurch wird eine genaue Platzierung des Herzklappenstents 10'" ermöglicht. Zur Fixierung des Herzklappenstents weist der Fixierschaft 47 an seinem distalen Ende einen Verbindungsabschnitt 49 auf, welcher mit einem entsprechenden, am proximalen Ende des zweiten Schafts 18"' angeordneten Verbindungsabschnitt 24' gekoppelt werden kann. Bei diesem Ausführungsbeispiel ist der zweite Schaft 18"' derart ausgebildet, dass er anders als bei den oben erläuterten Ausführungsbeispielen nicht in longitudinaler Richtung verschoben werden kann.

### Bezugszeichenliste

- 10, 10', 10", 10'": Herzklappenstent
- 11: Grundstruktur
- 12: Klappensegel
- 13: proximales Ende der Grundstruktur 11
- 14: distales Ende der Grundstruktur 11
- 15: Ring
- 16, 16': erster Schaft
- 17: konischer Abschnitt des ersten Schafts 16
- 18, 18', 18", 18"": zweiter Schaft
- 19: konischer Abschnitt des zweiten Schafts 18
- 20: erster Verbindungsabschnitt des ersten Schafts 16, 16'
- 21: Verbindungsabschnitt des zweiten Teils 32b des Innenschafts
- 23: zweiter Verbindungsabschnitt des ersten Schafts 16'
- 24, 24': Verbindungsabschnitt des zweiten Schafts 18', 18", 18"'
- 30, 30', 30": Katheter
- 32, 32': Innenschaft
- 32a: erster Teil des Innenschafts
- 32b: zweiter Teil des Innenschafts
- 34: erster Verbindungsabschnitt des Innenschafts 32
- 36,36': Außenschaft
- 37: konischer Abschnitt des zweiten Teils 32b des Innenschafts 32
- 39: Verbindungsabschnitt des Außenschafts 36'
- 40: Deckel
- 41: vorstehender Ring
- 43: Verbindungsabschnitt des Deckels 40
- 45: Pin
- 47: Fixierschaft
- 49: Verbindungsabschnitt des Fixierschafts 47

## Patentansprüche

1. Implantat, insbesondere eine intraluminale Endoprothese, mit einer durchbrochenen, hohlzylinder- und/oder hohlkegelförmigen Grundstruktur (11), wobei die Grundstruktur (11) einen komprimierten Zustand oder einen expandierten Zustand einnehmen kann, **dadurch gekennzeichnet, dass** zusätzlich ein erster rohrförmiger Schaft (16, 16') vorgesehen ist, auf dessen Außenseite die Grundstruktur (11) zumindest abschnittsweise im komprimierten Zustand angeordnet ist, wobei der erste Schaft (16, 16') einen ersten Verbindungsabschnitt (20, 23) aufweist, mit welchem der erste Schaft (16, 16') mit dem Innenschaft (32, 32', 32a, 32b) eines Katheters (30, 30', 30") verbindbar ist.

2. Implantat nach Anspruch 1, **dadurch gekennzeichnet, dass** ein zweiter, zumindest abschnittsweise rohrförmigen Schaft (18, 18', 18", 18"') vorgesehen ist, der die Grundstruktur (11) umgebend angeordnet ist, vorzugsweise an ihrem proximalen Ende (13).

3. Implantat nach Anspruch 2, **dadurch gekennzeichnet, dass** zusätzlich Mittel zur Fixierung des zweiten Schafts (18, 18', 18", 18"') in proximaler Richtung vorgesehen sind, beispielsweise ein proximal angeordneter Deckel (40) oder ein am proximalen Ende des zweiten Schafts angeordneter, in radialer Richtung vorstehender Pin (45).

4. Implantat nach einem der Ansprüche 2 bis 3, **dadurch gekennzeichnet, dass** der zweite Schaft (18, 18', 18", 18"') einen konischen Abschnitt (19) aufweist, der vorzugsweise am distalen Ende des zweiten Schafts (18, 18', 18", 18"') angeordnet ist.

5. Implantat nach einem der Ansprüche 2 bis 4, **dadurch gekennzeichnet, dass** der zweite Schaft (18, 18', 18", 18"') einen Verbindungsabschnitt (24, 24') aufweist, mit dem der zweite Schaft (18, 18', 18", 18"') mit einem Außenschaft (36, 36') oder einem Fixierschaft (47) des Katheters (30, 30', 30") verbindbar ist.

6. Implantat nach einem der vorhergehenden Ansprüche, **dadurch gekennzeichnet, dass** der erste Schaft (16, 16'), vorzugsweise an seinem distalen Ende (14), einen zweiten Verbindungsabschnitt (23) aufweist.

7. System zum Einbringen eines Implantats, vorzugsweise einer intraluminalen Endoprothese, in einen Körperhohlraum bestehend aus einem Implantat (10, 10', 10", 10'") nach einem der vorhergehenden Ansprüche und einem Katheter (30, 30', 30"), wobei der Innenschaft (32, 32') des Katheters (30, 30', 30") einen, vorzugsweise an seinem distalen Ende angeordneten Verbindungsabschnitt (34) aufweist, mit dem der Innenschaft (32, 32') mit dem ersten Schaft (16, 16') des Implantats (10, 10', 10", 10"') verbindbar ist.

8. System nach Anspruch 7, **dadurch gekennzeichnet, dass** der Außenschaft (36, 36') des Katheters (30, 30', 30") oder der Fixierschaft (47) des Katheters (30, 30', 30") jeweils einen, vorzugsweise an dem jeweiligen distalen Ende angeordneten, Verbindungsabschnitt (39, 49) aufweist, mit dem der Außenschaft (36, 36') bzw. der Fixierschaft (47) mit dem zweiten Schaft (18, 18', 18", 18"') des Implantats (10, 10', 10", 10'") verbindbar ist.

9. System nach Anspruch 7 oder 8, **dadurch gekennzeichnet, dass** der Innenschaft (32, 32') des Katheters (30, 30', 30") zweiteilig ausgebildet ist, wobei vorzugsweise der erste Teil (32a) durch einen proximalen Abschnitt und der zweite Teil (32b) durch einen distalen Abschnitt gebildet ist.

10. Verfahren zur Herstellung eines Systems nach einem der Ansprüche 7 bis 9, **dadurch gekennzeichnet, dass** vor dem Einbringen des Systems in einen Körperhohlraum eines Menschen oder Tiers der erste Verbindungsabschnitt (20) des ersten Schafts (16, 16') mit dem gegenüber liegenden Verbindungsabschnitt (21, 34) des Innenschafts (32, 32', 32a, 32b) des Katheters (30, 30', 30") verbunden wird.

11. Verfahren zur Herstellung eines Systems nach einem der Ansprüche 7 bis 9, **dadurch gekennzeichnet, dass** vor dem Einbringen des Systems in einen Körperhohlraum eines Menschen oder Tiers und bei einer zweiteiligen Ausbildung des Innenschafts (32a, 32b) des Katheters (30, 30', 30") der erste Verbindungsabschnitt (20) des ersten Schafts (16') mit dem gegenüber liegenden Verbindungsabschnitt (34) des ersten Teils des Innenschafts (32a) des Katheters (30, 30', 30") und der zweite Verbindungsabschnitt (23) des ersten Schafts (16') mit dem gegenüber liegenden Verbindungsabschnitt (21) des zweiten Teils des Innenschafts (32b) des Katheters (30, 30', 30") verbunden wird.

12. Verfahren nach einem der Ansprüche 10 oder 11, **dadurch gekennzeichnet, dass** der Verbindungsabschnitt (24, 24') des zweiten Schafts (18, 18', 18", 18"') mit dem gegenüber liegenden Verbindungsabschnitt (39) des Außenschafts (36') des Katheters (30, 30', 30") oder mit dem gegenüber liegenden Verbindungsabschnitt (49) des Fixierschafts (47) des Katheters (30, 30', 30") verbunden wird.
